# EUROPEAN PATENT SPECIFICATION

(11) **EP 2 728 325 B1**
(45) Date of publication and mention of the grant of the patent: **10.01.2018**
(21) Application number: 12194310.4
(22) Date of filing: 26.11.2012
(51) Int. Cl.: G01J 5/02

(54) **Probe cover for ear thermometer**
Sondenabdeckung für Ohrenthermometer
Couvercle de sonde pour thermomètre auriculaire

(30) Priority: 30.10.2012 TW 101140026
(43) Date of publication of application: 07.05.2014
(73) Proprietor: Actherm Inc., Hsinchu 30078 (TW)
(72) Inventor: Chen, Min-Ying, 303 Hsinchu County (TW)
(74) Representative: Lang, Christian

(56) References cited:
- CN-Y- 2 375 956
- US-A- 3 949 740
- US-B1- 6 647 284

## Description

### BACKGROUND OF THE INVENTION

### 1. Field of the Invention

The present invention relates to a probe cover for ear thermometers. By installing several elastic depresses at the open end of the probe cover for an ear thermometer, the probe cover for ear thermometers can easily cover the probe of the ear thermometer by the elasticity of the elastic depresses when the probe cover is installed on the ear thermometer so as to avoid the damage of the over extension.

### 2. Description of the Prior Art

According to the progress and the development of the technology, there are many body temperature measurement methods different from the conventional mercury thermometer gradually developed, such as ear thermometers, forehead thermometers and so on. The ear thermometer has become a popular body temperature measure method because of the advantages of fast measurement, high accuracy, easy operation and so on.

The measure method of the ear thermometer is to input a probe of the ear thermometer into the ear canal of the patient and determine the body temperature by detecting the electromagnetic wave irradiated from the human body. By considering the heath safety, this kind of measuring method is required to directly contact the human body, so there is a probe cover used to house the probe before measuring by the ear thermometer and the probe cover is released and discarded after measuring the body temperature.

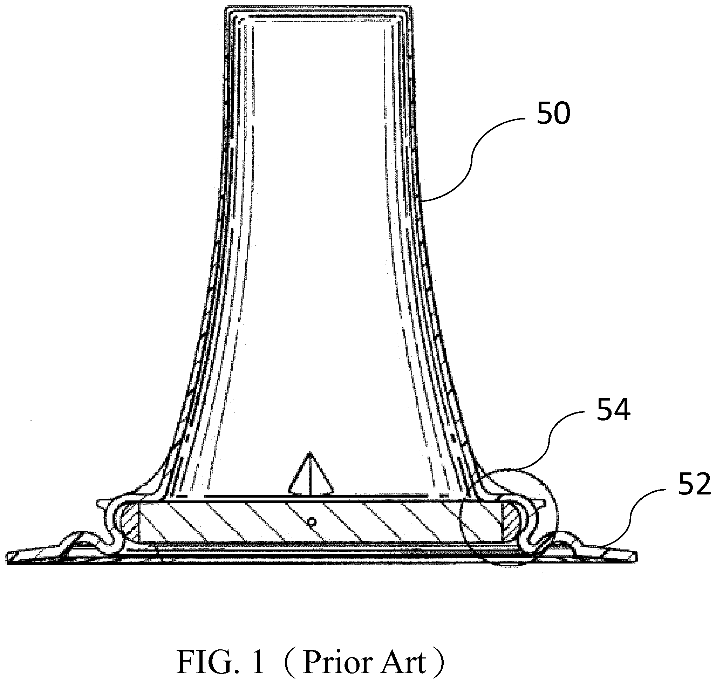

The conventional probe cover for ear thermometers is shown in FIG. 1 and includes a tapered main body 50 and a flange 52 extended from the bottom of the tapered main body 50. The tapered main body 50 or the flange 52 usually includes a protruding portion 54 configured to couple with an annular structure of the ear thermometer so as to fix the probe cover on the probe of the ear thermometer. However, the fixing method is required to push by the user when installing the probe cover and a probe cover releasing structure is required to release the probe cover from the probe and it is very inconvenient. The protruding portion 54 of the probe cover for the ear thermometer could be damaged because of over extension when the protruding portion 54 is used.

Therefore, according to the problems above, the ear thermometer needs a probe cover, which can be easily and firmly installed when the ear thermometer is used, and won't be damaged because of over extension.

As disclosed in U.S. Patent No. 3,949,740, a speculum (probe cover) for an ear thermometer comprises the general shape of a funnel with a first hollow, generally frustoconical base section having a relatively shallow taper. An enlarged bead is formed around the relatively larger open end of the base section. The base section blends gradually into a second hollow generally frustoconical nose or tip section having a relatively steep taper. The plastic of which the speculum is formed must be sufficiently elastically stretchable so that the stretching of the wall as it passes over the enlargement of the ear thermometer does not exceed the fast tensile strength of the plastic. When the speculum is installed on the ear thermometer, it remains in place until manually dislodged. In order to remove the speculum, all one needs to do is press forwardly on the bead and the speculum pops off readily.

From Chinese utility model CN 2 375 956 Y and US patent US 6 647 284 B1 further probe covers for ear thermometers are known, whereat according to the latter a flange-recess-connection is provided between the base of the probe cover and the probe of the ear thermometer in order to securely fix the probe cover on the probe.

### SUMMARY OF THE INVENTION

An object of the present invention is to provide a probe cover for ear thermometer, and the probe cover for ear thermometers is extendable. The probe cover for ear thermometer can be easily and firmly installed and won't be damaged because of over extending.

In order to achieve said object, the present invention provides a probe cover for an ear thermometer with the features of claim 1. Further embodiments of the probe cover according to the invention are subject-matter of the dependent claims. The probe cover for an ear thermometer is utilized for housing a probe of the ear thermometer and a bottom of the probe including an annular component, the probe cover for ear thermometers including a tapered main body with a hollow structure, the tapered main body including a first open end and a close end corresponding to the first open end, a diameter of the hollow structure is gradually shrunk from the first open end to the close end and the probe cover for ear thermometers includes an annular elastic portion, a flange, and a plurality of elastic recesses. The annular elastic portion is disposed adjacent to the open end to form a second open end, the second open end is an annular shape and the annular elastic portion includes an annular inner surface and an annular outer surface. The flange extends from the second open end and the flange includes a top surface and a bottom surface corresponding to the top surface and the top surface is faced to the close end. The elastic recesses are formed at the second open end, which is an intersection between the flange and the annular elastic portion. The gaps are formed at the elastic recess of the annular inner surface and the bottom surface of the flange and some protrusions are formed at the elastic recesses of the annular outer surface.

By using the probe cover for ear thermometers in the present invention, the annular open end of the probe cover for ear thermometers won't be damaged when it is extended because of the elastic concave. Because of the extension ability of the annular elastic portion, the probe cover for ear thermometers can be firmly installed on the probe of the ear thermometer.

The above-mentioned description of the present invention can be best understood by referring to the following detailed description of the preferred embodiments and the accompanying drawings.

### BRIEF DESCRIPTION OF THE DRAWINGS

FIG. 1 is a view illustrating a probe cover for ear thermometers according to prior art;
FIG. 2 is a side view illustrating a probe cover for ear thermometers according to one embodiment of the present invention;
FIG. 3 is a top view illustrating the probe cover for ear thermometers according to the same embodiment of the present invention;
FIG. 4 is a view illustrating a bore diameter comparing between the probe cover for ear thermometers and the probe according to the same embodiment of the present invention;
FIG. 5A is a view illustrating an annular open end of the probe cover for ear thermometers according to the same embodiment of the present invention before extending;
FIG. 5B is a view illustrating an annular open end of the probe cover for ear thermometers according to the same embodiment of the present invention after extending; and
FIG. 6 is a view illustrating the probe cover for ear thermometers installed on the probe according to the same embodiment of the present invention.

### DESCRIPTION OF THE PREFERRED EMBODIMENT

In the following detailed description of the preferred embodiments, reference is made to the accompanying drawings which form a part hereof, and as shown by way of illustration specific embodiments in which the invention may be practiced. In this regard, directional terminology, such as "top," "bottom," "front," "back," "left," "right," "inside," "outside," "side," etc., is used with reference to the orientation of the Figure(s) being described. As such, the directional terminology is used for purposes of illustration and is in no way limiting the present invention.

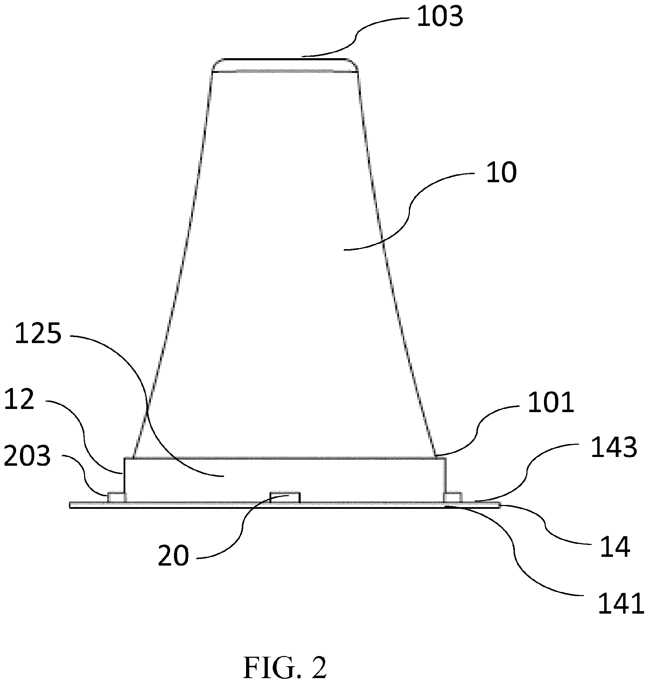

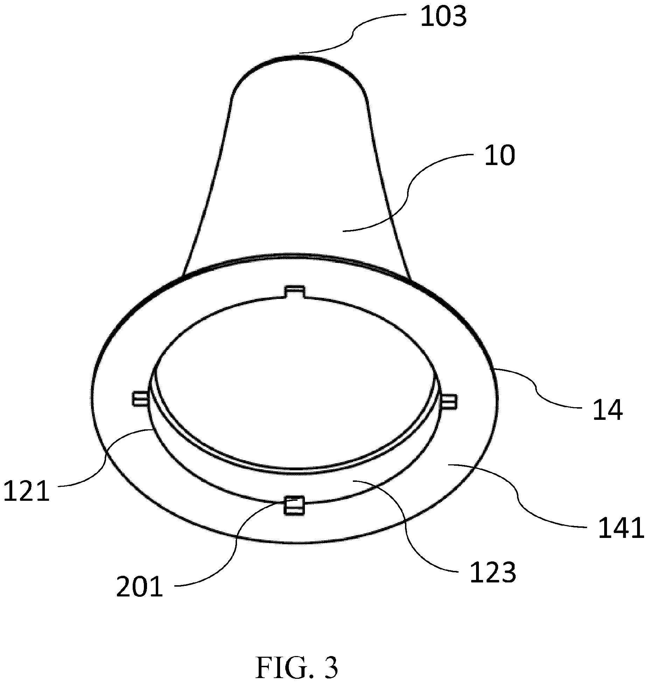

At first, please refer to FIG. 2 and FIG. 3, which are a side view and a top view of a probe cover for ear thermometer in one embodiment of the present invention. As shown in FIG. 2 and FIG. 3, the probe cover 1 for ear thermometers includes a tapered main body 10 with a hollow structure, an annular elastic portion 12, a flange 14 and several elastic recesses 20.

Specifically, the tapered main body 10 includes an opening end 101 and a close end 103 corresponding to the opening end 101. The diameter of the hollow structure is gradually decreased from the open end 101 to the close end 103 and the hollow structure of the tapered main body 10 is a short cone, which a top end thereof is cut. The annular elastic portion 12 is disposed near the open end 121 and formed an annular open end 121 and the annular elastic portion 12 includes an annular inner surface 123 and an annular outer surface 125. The flange 14 is extended from the annular open end 121 to outside and the flange 14 includes a bottom surface 141, which is faced to the same direction as the annular open end 121 and a top surface 143, which is faced to the same direction as the close end 103 and corresponding to the bottom end 141. The top surface is faced to the close end 103. The elastic recesses 20 are respectively and sequentially disposed with the same distance in the annular open end 121, which is at a junction of the flange 14 and the annular elastic portion 12. The elastic recesses 20 are respectively formed as gaps 201 between the annular inner surface 123 and the bottom surface 141 of the flange 14. The elastic outer surface 125 is formed as protruding portion 203 at the elastic recesses 20. The number of the elastic recesses is 4 in the present embodiment and the shape of the gaps 201 formed in the elastic recesses 20 at the junction of the annular inner surface 123 and the bottom surface 141 of the flange 14 is rectangular. The elastic recesses 20 are symmetrically disposed by pair. However, the number and the shape of the elastic recesses in the present invention are not limited herein. For example, the shape can be polygon and the number of the elastic recess can be only one. In addition, the tapered main body 10, the annular elastic portion 12, the flange 14 and the elastic recess 20 are formed integrally and the material thereof is high polymer, but it is not limited in the present invention.

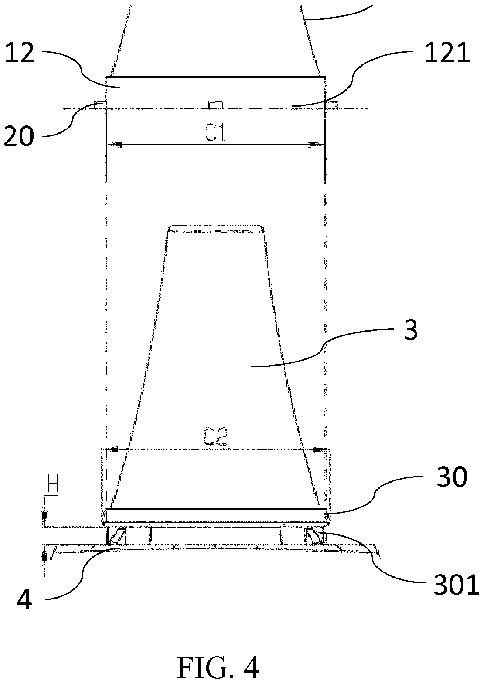

Now, please refer to FIG. 4, which is a bore diameter comparing view illustrating the probe cover for ear thermometers and the probe of the ear thermometer in the embodiment of the present invention. As shown in FIG. 4, when measuring the body temperature, the probe cover 1 for ear thermometers is housing on the probe 3 of the ear thermometer body 4 and there is an annular flange 30 in the surrounding area of the bottom end of the probe 3. The bore diameter of the annular open end 12 in the present embodiment is C₁ and the bore diameter of the annular flange 30 of the probe 3 is C₂. In order to fix the probe cover 1 on the probe 3, C₂ must be larger than C₁. The annular open end 121 of the probe cover 1 for ear thermometers must be elastic and can be extended properly when the probe cover 1 for ear thermometer is fixed on the probe 3 so as to be successfully installed.

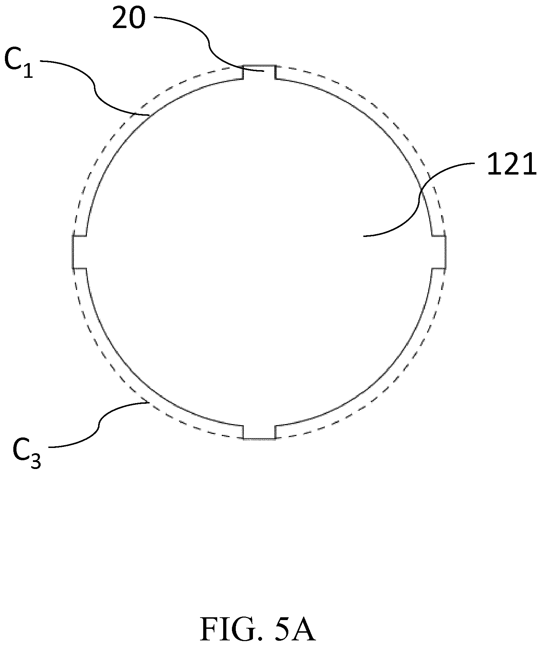

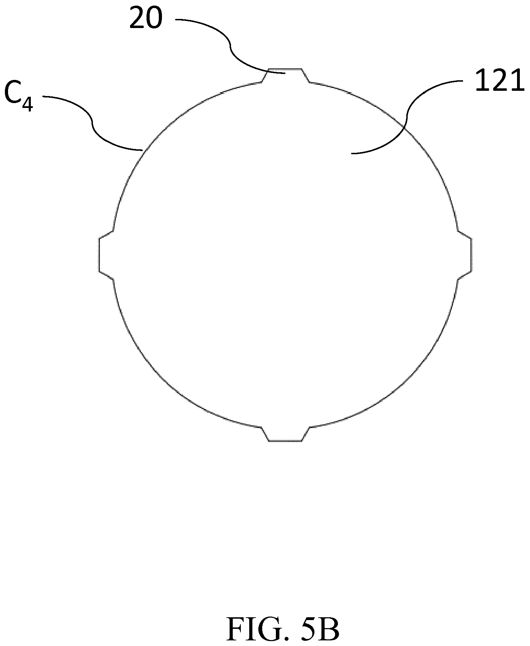

Subsequently, please refer to FIG. 5A and FIG. 5B, which are views illustrating the extension of the annular open end of the probe cover for ear thermometers in the present embodiment. First of all, as shown in FIG. 5A, before the annular open end 121 is extended, the bore diameter thereof is C₁, and the bore diameter of the external dot lines C₃ is larger than C₁ in accordance with FIG. 5A, the C₃ is represented the limit of the extension of the annular open end 121. Thereafter, please refer to FIG. 4 and FIG. 5B at the same time, as the probe cover 1 for ear thermometers is housing the probe 3, the annular flange 30 of the probe 3 will contact the annular open end 121. Because the bore diameter C₂ of the annular flange 30 is larger than the bore diameter C₁ of the annular open end 121, the annular open end 121 is extended. Because the elastic recess 20 is disposed in the annular open end 121, the annular open end 121 will be extended because of the elasticity thereof in accordance with the elastic recess 20 in the surrounding area of the annular open end 121. The bore diameter C₁ is extended for the annular flange 30 passing through the bore diameter C₄ of the annular open end 121. Therefore, the probe cover 1 for ear thermometers is able to house the probe 3 successfully. At the same time, after the annular flange 30 is passing through the annular open end 121, the annular open end 121 is back to the original bore diameter C₁ because of the spring reversion force of the elastic recess 20 and stuck on the surface of the probe 3. Therefore, the annular open end 121 won't be over extended, which would cause the possibility of the damage of the probe cover 1 for ear thermometer when the probe cover 1 is in use, to reduce an abrasion of the probe 3 of the ear thermometer.

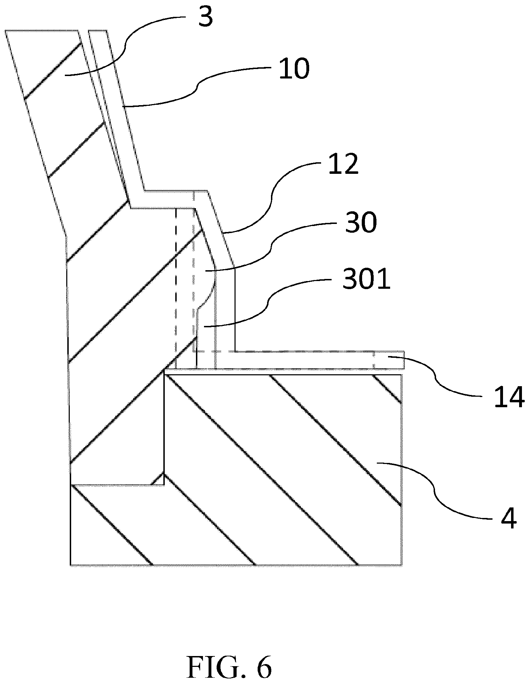

Now, please refer to FIG. 6, which is a view illustrating that the probe cover is installed on the ear thermometer according to the previous embodiment of the present invention. As shown in FIG. 6, by implementing the extension of the elastic recess 20, the probe cover 1 for ear thermometers can easily and fastly house on the probe 3. In addition, please refer to FIG. 4 and FIG. 6 in conjunction, there is a distance H (as shown in FIG. 4) reserved between the annular flange 30 on the probe 3 of the ear thermometer and the main body 4 of the ear thermometer. In other words, there is a gap 301 below the annular flange 30 and an outer circumference thereof is smaller than the bore diameter C₂ of the annular flange 30. Therefore, when the annular flange 30 is passing through the annular open end 121 because of the extension (the dot line in FIG. 6 in a non-extension condition) of the annular open end 121, the annular open end 121 will be shrunk because of the elasticity and the spring reversion force of the elastic recess 20 to house the surface of the gap 301 below the annular flange 30 after passing through the annular open end 121 and it is in a condition that the annular elastic portion 12 houses the annular flange 30. The probe cover for ear thermometers is not easily loosed.

## Claims

1. A probe cover (1) for an ear thermometer, which is utilized for housing a probe (3) of the ear thermometer and a bottom of the probe (3) including an annular component (30), the probe cover (1) including a tapered main body (10) with a hollow structure, the tapered main body (10) including a first open end (101) and a closed end (103) corresponding to the first open end (101), a diameter of the hollow structure is gradually shrunk from the first open end (101) to the closed end (103) and the probe cover (1) comprises
an annular elastic portion (12) disposed adjacent to the first open end (101) to form a second open end (121), the second open end (121) being an annular shape and the annular elastic portion (12) includes an annular inner surface (123) and an annular outer surface (125);
a flange (14) extending from the second open end (121) and the flange (14) including a top surface (143) and a bottom surface (141) corresponding to the top surface (143) and the top surface (143) being faced to the closed end (103); **characterised in that**
the probe cover (1) further comprises a plurality of elastic recesses (20) equally formed at the second open end (121), which is an intersection between the flange (14) and the annular inner surface (123), with a distance and gaps (201) being formed at the elastic recesses (20) of the annular inner surface (123) and the bottom surface (141) of the flange (14) and protrusions (203) being formed on the annular outer surface (125) at positions corresponding to the elastic recesses (20).

2. The probe cover (1) for ear thermometer according to claim 1, wherein the tapered main body (10), the annular elastic portion (12), the flange (14) and the elastic recesses (20) are formed integrally.

3. The probe cover (1) for ear thermometer according to claim 1, wherein the hollow structure of the tapered main body (10) is a short cone with a cut top end.

4. The probe cover (1) for ear thermometer according to claim 1, wherein the elastic recesses (20) are disposed by pair.

5. The probe cover (1) for ear thermometer according to claim 1, wherein the shape of the gaps (201) formed at the elastic recess (20) of the bottom surface (141) of the flange (14) is rectangular.

6. The probe cover (1) for ear thermometer according to claim 1, wherein the shape of the gaps (201) formed at the elastic recess (20) of the bottom surface (141) of the flange (14) is polygonal.

7. The probe cover (1) for ear thermometer according to claim 1, wherein a material of the probe cover (1) for ear thermometer is high polymer.

## Patentansprüche

1. Messsondenabdeckung (1) für ein Ohrthermometer, welche zur Aufnahme einer Messsonde (3) des Ohrthermometers verwendet wird, wobei eine Unterseite der Messsonde (3) ein ringförmiges Element (30) aufweist, wobei die Messsondenabdeckung (1) einen konischen Grundkörper (10) mit einer Hohlstruktur umfasst, wobei der konische Grundkörper (10) ein erstes offenes Ende (101) und ein mit dem ersten offenen Ende (101) korrespondierendes geschlossenes Ende (103) aufweist, wobei ein Durchmesser der Hohlstruktur von dem ersten offenen Ende (101) zu dem geschlossenen Ende (103) allmählich kleiner wird, und wobei die Messsondenabdeckung (1) umfasst
einen ringförmigen elastischen Abschnitt (12), der angrenzend an das erste offene Ende (101) angeordnet ist, um ein zweites offenes Ende (121) auszubilden, wobei das zweite offene Ende (121) eine ringförmige Gestalt hat, und wobei der ringförmige elastische Abschnitt (12) eine ringförmige Innenfläche (123) und eine ringförmige Außenfläche (125) aufweist,
einen Flansch (14), der sich von dem zweiten offenen Ende (121) erstreckt, wobei der Flansch (14) eine obere Fläche (143) und eine mit der oberen Fläche (143) korrespondierende untere Fläche (141) aufweist, wobei die obere Fläche (143) dem geschlossenen Ende (103) zugewandt ist, **dadurch gekennzeichnet, dass**
die Messsondenabdeckung (1) ferner eine Mehrzahl von elastischen Aussparungen (20) umfasst, die gleichmäßig an dem zweiten offenen Ende (121) ausgebildet sind, welches eine Schnittfläche zwischen dem Flansch (14) und der ringförmigen Innenfläche (123) ist, wobei ein Abstand und Lücken (201) an den elastischen Aussparungen (20) der ringförmigen Innenfläche (123) und der unteren Fläche (141) des Flansches (14) ausgebildet sind, und wobei Vorsprünge (203) an der ringförmigen Außenfläche (125) an mit den elastischen Aussparungen (20) korrespondierenden Positionen ausgebildet sind.

2. Messsondenabdeckung (1) für ein Ohrthermometer nach Anspruch 1, bei welcher der konische Grundkörper (10), der ringförmige elastische Abschnitt (12), der Flansch (14) und die elastischen Aussparungen (20) integral ausgebildet sind.

3. Messsondenabdeckung (1) für ein Ohrthermometer nach Anspruch 1, bei welcher die Hohlstruktur des konischen Grundkörpers (10) ein kurzer Kegel mit einem abgeschnittenen oberen Ende ist.

4. Messsondenabdeckung (1) für ein Ohrthermometer nach Anspruch 1, bei welcher die elastischen Aussparungen (20) paarweise angeordnet sind.

5. Messsondenabdeckung (1) für ein Ohrthermometer nach Anspruch 1, bei welcher die Form der Lücken (201), die an den elastischen Aussparungen (20) der unteren Fläche (141) des Flansches (14) ausgebildet sind, rechteckig ist.

6. Messsondenabdeckung (1) für ein Ohrthermometer nach Anspruch 1, bei welcher die Form der Lücken (201), die an den elastischen Aussparungen (20) der unteren Fläche (141) des Flansches (14) ausgebildet sind, polygonal ist.

7. Messsondenabdeckung (1) für ein Ohrthermometer nach Anspruch 1, bei welcher ein Material der Messsondenabdeckung (1) für das Ohrthermometer hochpolymer ist.

## Revendications

1. Couvercle de sonde (1) pour thermomètre auriculaire qui est utilisé pour loger une sonde (3) du thermomètre auriculaire et une partie inférieure de la sonde (3) qui comprend un composant annulaire (30), le couvercle de sonde (1) comprenant un corps principal effilé (10) avec une structure creuse, le corps principal effilé (10) comprenant une première extrémité ouverte (101) et une extrémité fermée (103) correspondant à la première extrémité ouverte (101), un diamètre de la structure creuse étant diminué graduellement de la première extrémité ouverte (101) à l'extrémité fermée (103) et le couvercle de sonde (1) comprenant :
une portion élastique annulaire (12) disposée adjacente à la première extrémité ouverte (101) pour former une seconde extrémité ouverte (121), la seconde extrémité ouverte (121) étant une forme annulaire et la portion élastique annulaire (12) comprenant une surface intérieure annulaire (123) et une surface extérieure annulaire (125),
une bride (14), qui s'étend à partir de la seconde extrémité ouverte (121) et de la bride (14), comprenant une surface de dessus (143) et une surface inférieure (141) correspondant à la surface de dessus (143) et la surface de dessus (143) faisant face à l'extrémité fermée (103),
**caractérisé en ce que**
le couvercle de sonde (1) comprend de plus une pluralité d'évidements élastiques (20) formés de manière égale au niveau de la seconde extrémité ouverte (121) qui est une intersection entre la bride (14) et la surface intérieure annulaire (123) avec une distance et des vides (201) qui sont formés au niveau des évidements élastiques (20) de la surface intérieure annulaire (123) et de la surface inférieure (141) de la bride (14) et des saillies (203) étant formées sur la surface extérieure annulaire (125) à des positions qui correspondent aux évidements élastiques (20).

2. Couvercle de sonde (1) pour thermomètre auriculaire selon la revendication 1, le corps principal effilé (10), la portion élastique annulaire (12), la bride (14) et les évidements élastiques (20) étant formés en une seule pièce.

3. Couvercle de sonde (1) pour thermomètre auriculaire selon la revendication 1, la structure creuse du corps principal effilé (10) étant un cône court avec une extrémité supérieure coupée.

4. Couvercle de sonde (1) pour thermomètre auriculaire selon la revendication 1 dans lequel les évidements élastiques (20) sont disposés par paire

5. Couvercle de sonde (1) pour thermomètre auriculaire selon la revendication 1 dans lequel les vides (201) formés au niveau de l'évidement élastique (20) de la surface inférieure (141) de la bride (14) sont rectangulaires.

6. Couvercle de sonde (1) pour thermomètre auriculaire selon la revendication 1 dans lequel la forme des vides (201) formés au niveau de l'évidement élastique (20) de la surface inférieure (141) de la bride (14) sont polygonaux.

7. Couvercle de sonde (1) pour thermomètre auriculaire selon la revendication 1 dans lequel un matériau du couvercle de sonde (1) pour le thermomètre auriculaire est un polymère élevé.
